# EUROPEAN PATENT APPLICATION

(11) **EP 3 123 961 A1**
(43) Date of publication of application: **01.02.2017**
(21) Application number: 16181911.5
(22) Date of filing: 29.07.2016
(51) Int. Cl.: A61B 17/115, A61B 90/00, A61B 17/11

(54) **INDICATOR SYSTEM OF A CIRCULAR STAPLER AND A CIRCULAR STAPLER**

(30) Priority: 31.07.2015 PL 41336415
(71) Applicant: Grena Limited, Brentford TW8 9HH (GB)
(72) Inventor: Brodaczewski, Wieslaw, 05-410 Józefów (PL); Decewicz, Andrzej, 05-410 Józefów (PL); Redosz, Radoslaw, 02-785 Warszawa (PL)
(74) Representative: Kondrat, Mariusz

(57) **Abstract**

The object of the invention is an indicator system of a circular stapler for connecting tissue fragments, which consists of an indicator, an axis shaft of the indicator placed in a body, a tendon system comprising a needle, a tendon, a bolt, support means; a lead screw, an indicator spring and a knob, characterised in that the indicator has the form of a half-sleeve with attached fastening means and indicating means, and moves in a slidably rotational manner on the axis shaft of the indicator as a result of rotating the knob, and consequently the tendon system, which causes the anvil to approach to the load; while the lead screw in a distal area has a projection constituting the finger of the impactor, wherein after reaching a gap of 1 mm between the anvil and the load, the finger of the impactor presses on the upper surface of the indicator, causing it to tilt and indicate the value of 1 mm in the window located on the opposite side of the finger of the impactor and a circular stapler comprising an indicator system.

## Description

The invention relates to an indicator system of a circular stapler used in surgical anastomosis, especially intended for intestinal connections, with the assembly connection performed using surgical staples. The invention also relates to a circular stapler comprising an indicator system.

Description in US2013193187 discloses a surgical device for stapling, comprising a grip assembly with an ignition trigger, a firing trigger, which may move between a first non-active position and a second active position; an elongated body extending beyond the end of the grip assembly; a head portion placed in the proximity of the distal portion of the elongated body and comprising an anvil assembly and a cover assembly, the anvil assembly is movable relative to the cover assembly between the positions: remote and near; and an indicator placed in mechanical cooperation with the grip assembly and may move between the first position and the second position in response to the movement of the retaining means towards its near position, and the indicator is movable between the second position and a third position, in response to the firing of the instrument. The grip assembly comprises a pawl and the ignition trigger is adapted to get in contact with the pawl when triggering the ignition is slid to the second position. The indicator comprises at least two different marks comprising the first indication, visible to the user when the indicator is in its second position, and the second sign visible to the user when the indicator is in the third position. The indicator is rotated relative to the immovable grip. The grip assembly comprises a window, a portion of the indicator visible through the window. The indicator is tilted towards its first position. The indicator is rotated in a first direction, from the first position towards the second position, and from its second position towards the third position. The grip assembly comprises a pawl, further comprising a sliding means movably connected to the indicator, the pawl comprising a support surface, adapted to engage a slidable element to cause another proximal movement of the slidable element in order to slide the indicator to the third position.

Document US2015/0083772 A1 discloses a surgical circular stapler, which has a body, a shaft, a stapling assembly, an motor, a cam assembly, and a firing assembly. Shaft/spindle extends distally from the body. The stapling assembly is attached to the distal end of the spindle. Longitudinal transposition of the firing assembly causes the stapling assembly to drive multiple staples in a roller system to provide two clearances of the tissue together. The stapling assembly may further lead the blade to sever the excess of the tissue inside the circular system of the staples. The motor is adapted to rotate the cam assembly. Rotation of the cam assembly results in a longitudinal transposition of the firing assembly. A single rotation of the cam assembly is adapted to drive the firing assembly from the proximal position to the distal position and again back to the proximal position.

In known solutions, the indicator cooperates mechanically with the grip assembly and may move in three positions. Moving the indicator is possible due to the movement of jaw elements. Unfortunately, these solutions are based on the cooperation of the indicator with multiple mechanical means, which may lead to the blocking of one of them or to producing friction that prevents proper functioning of the entire system.

The aim of the present invention was to develop a reliable indicator system by limiting the number of mechanical components.
The indicator system according to the invention cooperates only with a finger of the impactor. This allows to simplify the entire device and at the same time it makes it independent of the occurrence of technical defects of multiple means.
An indicator of a circular stapler according to the invention shows two values: 1mm and 1.5mm. These values show what will be, after firing/launching the stapler, the space between the load (in which surgical staples and the knife are placed) and the anvil (which closes the surgical staples).
The improvement is that at the value of 1mm, the knob is blocked by the lead screw. Once the knob is blocked, it is not possible to further reduce the gap between the anvil and the load. Thus, there occurs no crushing of the tissue to be closed. A gap of less than 1 mm is a highly undesirable phenomenon, as it would result in disadvantageous irreversible effects in the form of damage, crushing of the intestinal tissue to be connected.
On the lead screw there is a finger of the impactor which, when the knob is rotated, presses on the indicator means causing it to tilt. When the value of 1 mm is reached between the anvil and the load, the tilt of the indicator shows the value of 1 mm.
The indicator has only two positions (1 and 1.5 mm), with other embodiments including 3 positions.
The indicator according to the invention prevents the gap from being set below 1 mm. This is so because on the lead screw there is a blocking means, which prevents further rotation of the knob after reaching a gap of 1 mm.
The advantages of using the invention include the possibility of observing the value of the gap between the load and the anvil, which is not visible to the operator. The indicator system allows the knob to be blocked when a value of the gap is reached between the load and the anvil of 1 mm, which prevents crushing or tearing the tissue. Furthermore, the values shown by the indicator between 1.5 and 1 mm inform the operator that he approaches the value of 1 mm.

The essence of the invention is an indicator system of a circular stapler for connecting tissue fragments, which consists of an indicator, an axis shaft of the indicator placed in the body, a tendon system comprising a needle, a tendon, a bolt, support means; a lead screw, an indicator spring and a knob, characterised in that the indicator has the form of a half-sleeve with attached fastening means and indicating means, and moves in a slidably rotational manner on the axis shaft of the indicator as a result of rotating the knob, and consequently the tendon system, which causes the anvil to approach to the load; while the lead screw in a distal area has a projection constituting the finger of the impactor, wherein after reaching a gap of 1 mm between the anvil and the load, the finger of the impactor presses on the upper surface of the indicator, causing it to tilt and indicate the value of 1 mm in the window located on the opposite side of the finger of the impactor.
The invention is also related to a circular stapler for connecting tissue fragments, comprising a shared connecting unit placed on a body comprising a load connected to an anvil by means of a tendon system; a grip, an indicator, a knob and an impactor system characterised in that it comprises the indicator system described above.

The circular stapler is used in general surgery, thoracic surgery, bariatric surgery, in colon and rectum resection, for performing end-to-end, side-to-end and side-to-side connections throughout the gastrointestinal tract. Before the first use, the stapler should be washed from the residue of the preservative and sterilised in an autoclave. After each use, the stapler needs to be washed and sterilised, so that it can be used again. This applies only to the device itself, as the loads and the anvil are delivered in a sterile condition and are intended for single use. The sterile load needs to be screwed clockwise on the distal portion of the stapler. Preparing the stapler for use requires applying purse-string sutures at both ends to be connected. Then, using the knob clockwise, it is necessary to completely hide the needle in the body of the device. The anvil is inserted into the light of the organ and the purse string suture is tightened on the opening of the purse-string suture. Then, the circular stapler is inserted with the anvil disconnected into the second portion of the organ to be connected until the light is occluded. By rotating the knob counter-clockwise, it is necessary to completely remove the needle piercing the occluded light of the organ. In the next step, the tissue slides to the base of the needle exposing the suture ligature site. Then, the spindle of the anvil needs to be connected to the needle. For a proper connection, an audible click is required. Then, a space (a gap) is determined between the anvil and the load by rotating the knob clockwise. When the indicator shows the appropriate value (1 mm), the bolt unblocks the hook, and subsequently the grip. The stapler is ready for use. To perform the connection, the grip is pressed, in a strong and steady movement. Then, the grip needs to be released, and it returns to the initial position. Rotating the knob counter-clockwise by no more than 2 to 3 rotations will lead to distancing the anvil from the load. The stapler needs to be removed by gently pulling backwards in a swinging movement. After removing the disposable connecting unit it is necessary to check the integrity of the connection, while the tissue resected by the circular knife needs to be carefully verified whether it forms a full ring and whether it comprises all the layers throughout the perimeter.

The indicator system according to the invention consists of an indicator in the form of a half-sleeve with attached fastening means and indicating means, moving on an axis shaft of the indicator placed in a body, a tendon system, a lead screw with a finger of the impactor, an indicator spring and a knob 7.

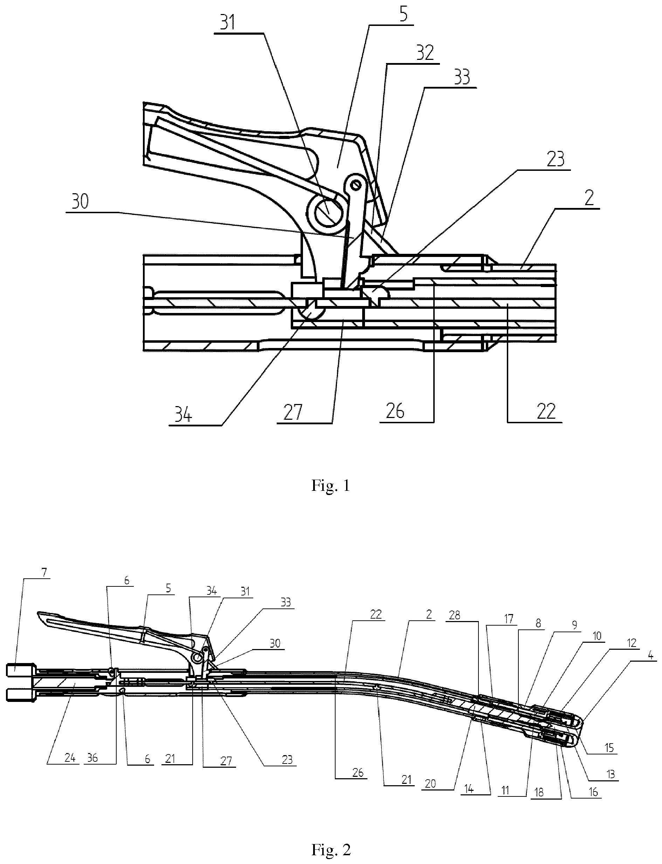

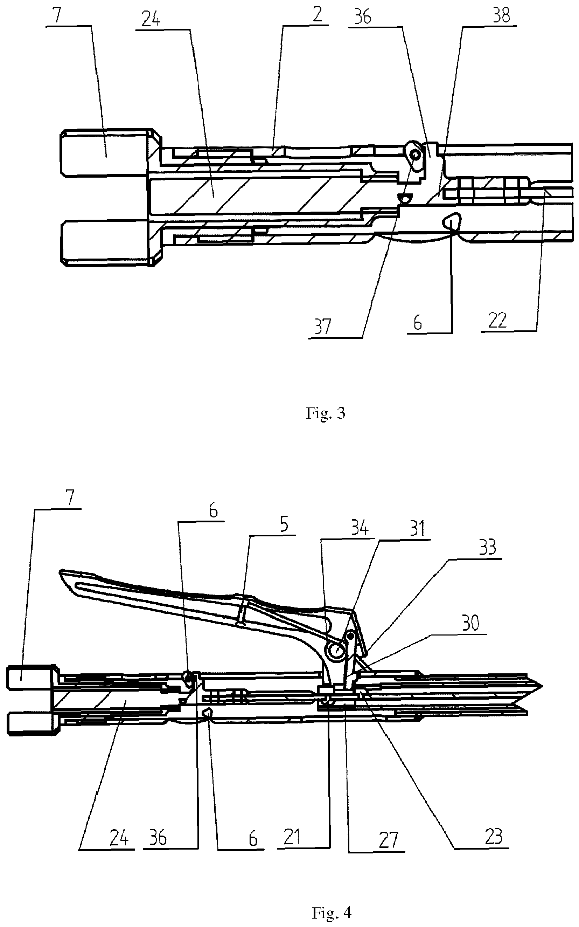

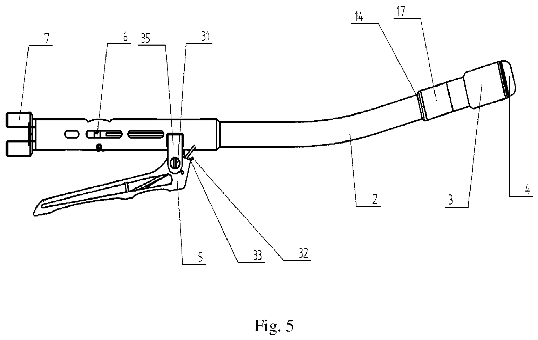

The object of the invention is shown in the embodiment and in the drawing, where fig. 1 shows a cross section of a fragment of the circular stapler including the grip system, fig. 2 shows a cross section of the circular stapler, fig. 3 shows a cross section of the circular stapler comprising the indicator system, fig. 4 shows a cross section of the circular stapler comprising the indicator system and the grip system, fig. 5 shows a perspective view of the circular stapler according to the invention.

The indicator system consists of an indicator 6 in the form of a half-sleeve with attached fastening means and indicating means, moving on an axis shaft of the indicator 37 placed in a body 2 of a tendon system 19 consisting of a needle 20, a tendon 22, a bolt 23, support means 34; a lead screw 24 with a finger of the impactor 36, an indicator spring 38 and a knob 7.

Rotating the knob 7 clockwise causes the movement of a tendon system 19 consisting of a needle 20, on which an anvil 4 is lockably mounted, a tendon 22, a bolt 23, support means 34 and a lead screw 24 with a finger of the impactor 36. Moving the tendon system towards the proximal end of the circular stapler causes the anvil to approach 4 the load 3, and between the anvil 4 and the load 3 a gap must be formed of at least 1 mm.

After reaching a distance between the anvil 4 and the load 3 of 1 mm , the finger of the impactor 36 in the form of a projection positioned on the lead screw 24 presses on the upper surface of the indicator 6, making it tilt and indicate the value of 1 mm in the window located on the opposite side of the finger of the impactor 36. This allows the operator to observe the size of the gap anvil/load. Rotating the knob 7 counter-clockwise causes the tendon system to distance 19 (of the anvil 4 from the load 3). This action also causes the finger of the impactor 36 to distance from the upper surface of the indicator 6, which due to the tightening of the indicator spring 38 gets back to the initial position.

## Claims

1. An indicator system of a circular stapler for connecting tissue fragments, which consists of an indicator, an axis shaft of the indicator placed in a body, a tendon system comprising a needle, a tendon, a bolt, support means; a lead screw, an indicator spring and a knob, **characterised in that** the indicator 6 has the form of a half-sleeve with attached fastening means and indicating means, and moves in a slidably rotational manner on the axis shaft of the indicator 37 as a result of rotating the knob 7, and consequently the tendon system 21, which causes the anvil 4 to approach the load 3; while the lead screw 24 in a distal area has a projection constituting the finger of the impactor 36, wherein after reaching a gap of 1 mm between the anvil 4 and the load 3, the finger of the impactor 36 presses on the upper surface of the indicator 6, causing it to tilt and indicate the value of 1 mm in the window located on the opposite side of the finger of the impactor 36.

2. A circular stapler for connecting tissue fragments, comprising a shared connecting unit placed on a body comprising a load connected to an anvil by means of a tendon system; a grip, an indicator, a knob and an impactor system **characterised in that** it comprises an indicator system described in claim 1.
